# EUROPEAN PATENT APPLICATION

(11) **EP 2 626 085 A1**
(43) Date of publication of application: **14.08.2013**
(21) Application number: 11830778.4
(22) Date of filing: 07.10.2011
(51) Int. Cl.: A61K 49/04

(54) **LIQUID PREPARATION FOR ORAL ADMINISTRATION WHICH CAN BE USED IN CT COLONOGRAPHY, AND COMPOSITION FOR IMAGING DIGESTIVE TRACT**

(30) Priority: 08.10.2010 US 391182 P
(71) Applicant: Ajinomoto Co., Inc., Tokyo 104-8315 (JP)
(72) Inventor: MOTOYAMA Takehiko, Tokyo 104-0042 (JP); NAGATA Koichi, Fukuroi-shi Shizuoka 437-1101 (JP); HONDA Tetsuro, Minamimatsuura-gun Nagasaki 857-4404 (JP)
(74) Representative: Nicholls, Kathryn Margaret
(86) International application number: PCT/JP2011/073236
(87) International publication number: WO 2012/046847

(57) **Abstract**

The present invention provides a liquid preparation for oral administration that is capable of reducing the amount of colonic irrigation solution taken during gastrointestinal pretreatment and decreasing the burden on the subject without sacrificing test accuracy and without prolonging the time required for pretreatment, and a composition for gastrointestinal radiography used to prepare the liquid preparation for oral administration. Namely, provided are a liquid preparation for oral administration taken during gastrointestinal imaging by CT colonography that comprises an iodine compound and a water-soluble polymer or saline laxative, wherein 300 mL to 1200 mL are taken on the day before CT colonography in a single administration or divided among several administrations, and a composition for gastrointestinal radiography comprising an iodine compound and a water-soluble polymer or saline laxative, wherein 300 mL to 1200 mL are taken in the form of an aqueous solution, having an iodine content of 3.5 mg/mL to 90 mg/mL prepared by dissolving in water, on the day before CT colonography in a single administration or divided among several administrations.

## Description

### TECHNICAL FIELD

The present invention relates to a liquid preparation for oral administration used in pretreatment for CT colonography, and a composition for gastrointestinal radiography used to prepare the liquid preparation for oral administration.

### BACKGROUND ART

Colorectal examination (CT colonography) performed by computed tomography (CT) is a test method that is becoming increasingly common due to the development of helical CT and multidetector CT. This test is already commonly used as a colon screening test in the U.S. and Europe. CT colonography consists of cleaning out solid stool matter in the large intestine by colonic irrigation, followed by performing CT imaging while dilating the lumen of the intestine with air, and then compiling and processing image data based on the slight difference in X-ray contrast between large intestinal tissue and air (see, for example, Non-Patent Document 1).

CT colonography does not require the insertion of an endoscope or barium in comparison with conventionally performed enema X-ray examinations and colonoscopy, and since examinations can be performed in a short period of time, the burden on the subject is considered to be decreased. In addition, since CT colonography allows digital data of two-dimensional (2D) images obtained by imaging to be reconfigured and displayed as three-dimensional (3D) images in the manner of virtual endoscopy and the like, the large intestine can be diagnosed from various angles. In addition, CT colonography enables the entire abdominal region, including the large intestine, to be imaged all at once in the short period of time it takes to hold one's breath, and lesions in organs other than the large intestine can be diagnosed simultaneously. Moreover, it does not require the need for the procedure of coating the intestinal mucosa with barium sulfate, which requires a sophisticated technique. CT colonography is expected to be applied in a wide range of applications in the future because of these advantages.

In comparison with colonoscopy that requires the visual removal of bowel residue and barium enema examinations that generate contrast in images by using a potent X-ray contrast medium in the form of barium sulfate, CT colonography consists of compiling and processing image data based on the slight difference in X-ray contrast between gastrointestinal tissue and air, thereby causing solid stool matter remaining in the large intestine to be easily mistaken for lesions. Consequently, it is necessary to remove intestinal contents during intestinal pretreatment in order to favorably depict two-dimensional images or three-dimensional images of the large intestine using multidetector CT such as multidetector-row CT (MDCT).

Colonic irrigation using an oral colonic irrigation agent is widely used for intestinal pretreatment. An example of the procedure for oral colonic irrigation consists of dissolving a powdered composition composed of electrolytes and the like in a fixed amount, such as about 2 liters, of water to prepare a colonic irrigation solution and then drinking the solution over the course of 2 to 4 hours. However, in cases of using a large amount of oral colonic irrigation solution to reduce the amount of residual stool, there is the problem of an increase in the amount of liquid residue. Since sites where residual water or intestinal juices have accumulated do not demonstrate contrast with gastrointestinal tissue, test images useful for diagnosis cannot be compiled. In other words, sites where residual water or intestinal juices have accumulated completely prevent the detection of lesions.

An example of a method for reducing areas where lesions cannot be detected by reducing the amount of liquid residue is disclosed in which a gastrointestinal prokinetic agent is used during oral colonic irrigation (see, for example, Patent Document 1). The amount of liquid residue in the intestinal tract can be reduced by taking the gastrointestinal prokinetic agent either before or after drinking the oral colonic irrigation solution or simultaneous to drinking the oral colonic irrigation solution.

In addition, the usefulness of electronic cleansing, which consists of removing residual water and accumulated liquid in the large intestine electronically by administering a contrast medium together with a colonic irrigation solution, is attracting attention. Since the use of a contrast medium causes residual water and areas of accumulated liquid residue to be labeled (tagged) resulting in an increase in their CT values (luminosity values), they can be distinguished from polyp lesions that are not tagged with contrast medium. For example, Patent Document 2 discloses a method that enables the entire tract of the large intestine to be examined by combining images of areas of liquid residue able to be depicted by the iodine-based water-soluble contrast medium with air images (gas images), and enables the locations of lesion sites to be confirmed while detecting colon cancer, by taking a composition for gastrointestinal radiography that combines an iodine-based water-soluble contrast medium and an oral colonic irrigation agent.

### [Prior Art Documents]

### [Patent Documents]

[Patent Document 1] International Publication No. WO 2004/087218
[Patent Document 2] Japanese Unexamined Patent Application, First Publication No. 2005-2006

### [Non-Patent Documents]

[Non-Patent Document 1] Liang, J.Z., ed., "3. Virtual Colonoscopy: An Alternative Approach to Examination of the Entire Colon", Inner Vision, 2001, Vol. 16, No. 10, pp. 40-44

### DISCLOSURE__OF THE INVENTION

### [Problems to be Solved by the Invention]

In this manner, although various types of proposals have already been made regarding intestinal pretreatment methods for CT colonography, it is desired to further enhance subject acceptance while maintaining the quality of test accuracy in order to be used as a test performed during group screening and general health examinations.
More specifically, conventional intestinal pretreatment methods for CT colonography require subjects to drink a large amount of colonic irrigation solution on the order of about 2 liters over the course of several hours in order to irrigate the colon, thereby placing a considerable burden on the subjects.

One of the problems with reducing the amount of colonic irrigation solution taken during pretreatment is that the contents of the intestine may not be able to be adequately tagged by the contrast medium. This problem can be resolved by performing pretreatment over the course of a long period of time such as for 2 or 3 days. This is because, even in the case the amount of oral colonic irrigation agent taken within the pretreatment time period is small or the oral colonic irrigation agent is not taken at all during that time period, all intestinal contents can be tagged with contrast medium by taking the contrast medium frequently at least two days prior to testing. However, a procedure that requires several days for pretreatment not only increases the burden on the subject, but also has a considerable effect on daily life in the case of performing for the purpose of group screening or general health examinations, and since there are also questions as to whether or not the proper pretreatment was performed as instructed, such a procedure is not practical. Therefore, there is a desire to adequately reduce the amount of oral colonic irrigation agent taken and minimize the number of days (time) required for pretreatment while ensuring subject acceptance.

An object of the present invention is to provide a liquid preparation for oral administration that is capable of reducing the amount of colonic irrigation solution taken during gastrointestinal pretreatment and decreasing the burden on the subject without sacrificing test accuracy and without prolonging the time required for pretreatment, and a composition for gastrointestinal radiography used to prepare the liquid preparation for oral administration.

### [Means for Solving the Problems]

As a result of conducting extensive studies to solve the aforementioned problems, the inventors of the present invention found that by taking a liquid preparation for oral administration containing an iodide compound, electrolytes and a water-soluble polymer on the day before CT colonography in an amount equal to roughly half the amount required for conventional colonic irrigation either in a single administration or divided among several administrations, subject acceptance can be dramatically improved and the depiction capacity of CT colonography can be maintained at a high level, thereby leading to completion of the present invention.

Namely, the present invention provides the following:
(1) a liquid preparation for oral administration taken during gastrointestinal imaging by CT colonography, comprising an iodine compound and a water-soluble polymer or saline laxative, wherein
   300 mL to 1200 mL are taken on the day before CT colonography in a single administration or divided among several administrations;
(2) the liquid preparation for oral administration described in (1) above, wherein 600 mL to 1200 mL are taken on the day before CT colonography in a single administration or divided among several administrations;
(3) the liquid preparation for oral administration described in (1) or (2) above, wherein the iodine compound is nonionic;
(4) The liquid preparation for oral administration described in any one of (1) to (3) above, wherein the water-soluble polymer is one or more selected from the group consisting of polyethylene glycol, polydextrose, dextran, dextrin, hydroxyethyl starch, gum arabic, pullulan, pectin, albumin and carboxymethyl cellulose;
(5) the liquid preparation for oral administration described in any one of (1) to (4) above, wherein osmotic pressure is 200 mOsm/L to 440 mOsm/L;
(6) the liquid preparation for oral administration described in any one of (1) to (5) above, further comprising one or more of sugars selected from the group consisting of sucrose, sorbitol, xylitol, erythritol, mannitol, trehalose, lactitol, lactulose, maltitol, palatinose, raffinose and glycerin;
(7) the liquid preparation for oral administration described in any one of (1) to (6) above, wherein the content of the iodine compound is 3.5 mg/mL to 90 mg/mL;
(8) the liquid preparation for oral administration described in any one of (1) to (7) above, further comprising one or more selected from the group consisting of dimethylpolysiloxane and gastrointestinal function accelerators;
(9) a composition for gastrointestinal radiography, comprising: an iodine compound and a water-soluble polymer or saline laxative, wherein
   300 mL to 1200 mL are taken in the form of an aqueous solution, having an iodine content of 3.5 mg/mL to 90 mg/mL prepared by dissolving in water, on the day before CT colonography in a single administration or divided among several administrations;
(10) the composition for gastrointestinal radiography described in (9) above, wherein 600 mL to 1200 mL are taken in the form of an aqueous solution, having an iodine content of 3.5 mg/mL to 90 mg/mL prepared by dissolving in water, on the day before CT colonography in a single administration or divided among several administrations;
(11) the composition for gastrointestinal radiography described in (9) or (10) above, wherein the iodine compound is nonionic;
(12) the composition for gastrointestinal radiography described in any one of (9) to (11) above, further comprising:
   electrolytes such that the osmotic pressure when dissolved in water is 200 mOsm/L to 440 mOsm/L, and
   one or more of water-soluble polymers selected from the group consisting of polyethylene glycol, polydextrose, dextran, dextrin, hydroxyethyl starch, gum arabic, pullulan, pectin, albumin and carboxymethyl cellulose, or
   one or more of saline laxatives selected from the group consisting of magnesium citrate, sodium dihydrogen phosphate and disodium hydrogen phosphate;
(13) the composition for gastrointestinal radiography described in any one of (9) to (12) above, further comprising one or more of compounds selected from the group consisting of dimethylpolysiloxane and gastrointestinal function accelerators;
(14) a pharmaceutical agent for pretreatment for CT colonography, comprising:
   combining an iodine compound and a water-soluble polymer or saline laxative, wherein 300 mL to 1200 mL are taken on the day before CT colonography in a single administration or divided among several administrations;
(15) the pharmaceutical agent for pretreatment for CT colonography described in (14) above, wherein 600 mL to 1200 mL are taken on the day before CT colonography in a single administration or divided among several administrations;
(16) a pharmaceutical agent for pretreatment for CT colonography, comprising: combining an oral colonic irrigation agent and an iodine-containing contrast agent, wherein 300 mL to 1200 mL are taken on the day before CT colonography in a single administration or divided among several administrations; and,
(17) the pharmaceutical agent for pretreatment for CT colonography described in (16) above, wherein 600 mL to 1200 mL are taken on the day before CT colonography in a single administration or divided among several administrations.

### [Effects of the Invention]

Use of the liquid preparation for oral administration of the present invention during pretreatment for CT colonography makes it possible to remarkably decrease the burden on the subject without sacrificing the quality of test accuracy.
In addition, use of the composition for gastrointestinal radiography of the present invention makes it possible to prepare the liquid preparation for oral administration of the present invention in the form of a preparation having superior convenience and ease of taking.

### BRIEF DESCRIPTION OF THE DRAWINGS

FIG. 1 is a photograph showing a three-dimensional image (endoscope-like image) within the transverse colon (T) of a Group A in Example 1.
FIG. 2 is a photograph showing a three-dimensional image (endoscope-like image) within the transverse colon (T) of a Group B in Example 1.
FIG. 3 is a photograph showing a three-dimensional image (endoscope-like image) within the transverse colon (T) of a Group B in Example 1.
FIG. 4 is a photograph showing a three-dimensional image (endoscope-like image) within the transverse colon (T) of a Group C in Example 1.
FIG. 5 is a photograph showing a three-dimensional image (endoscope-like image) within the transverse colon (T) of a Group D in Example 1.

### BEST MODE FOR OUT THE INVENTION

In the case the amount of oral colonic irrigation agent taken during pretreatment for CT colonography is inadequate, the cleansing and removal of intestinal contents such as solid stool matter becomes inadequate thereby resulting in poor test accuracy. Consequently, in the case of conventional pretreatment, the objective is to reduce the amount of solid stool matter and the like by cleansing the intestine by drinking a large amount of colonic irrigation solution over the course of several hours.

In contrast, the inventors of the present invention found that, although solid residue is to be eliminated from images of the inside of the intestine and it is preferable that such solid residue be removed, even if solid residue or residue in which solid residue has changed to a liquid or gelatinous state remains in the intestine, high depiction capacity comparable to that of the prior art can be maintained provided the residue is favorably tagged with contrast medium, and that the amount of solid residue remaining in the intestine is effectively reduced by taking a liquid preparation, obtained by containing an iodine-based water-soluble contrast medium in an oral colonic irrigation agent containing electrolytes and a water-soluble polymer, on the day before CT colonography in an amount equal to roughly half the amount used during conventional colonic irrigation.

It is difficult to distinguish between solid residue and polyp lesions in three-dimensional images such as virtual endoscopic images. In order to identify whether an image contains solid residue or a polyp lesion, it is necessary to confirm the internal characteristics of all protrusions observed in a three-dimensional image with a two-dimensional image (such as an axial image). Although the interior of solid residue demonstrates a high CT value since it is tagged with an oral contrast medium, polyp lesions normally demonstrate low CT values of 100 HU or less. In addition, although small pockets of gas are also present in the interior of solid residue in many cases, polyp lesions do not contain such gas pockets. Thus, solid residue and polyp lesions can be distinguished by two-dimensional images.

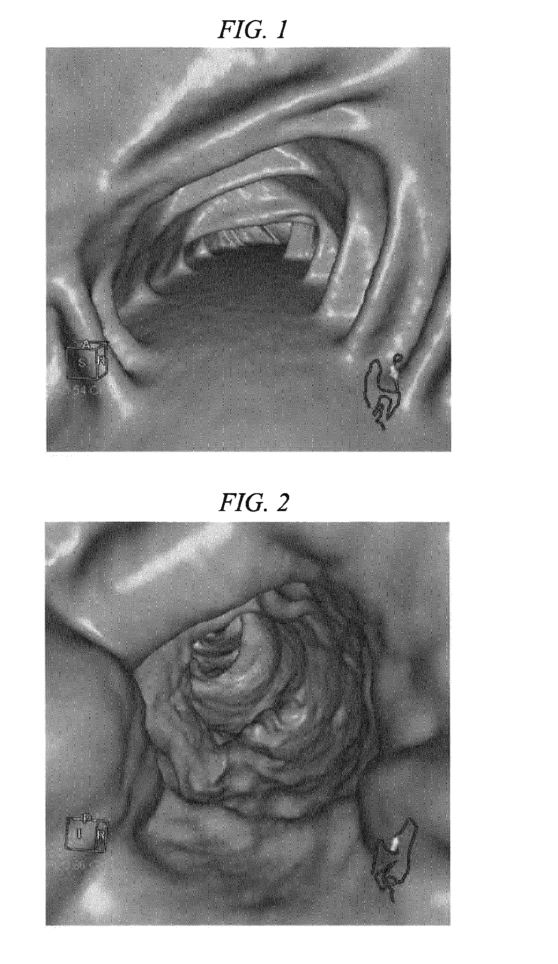

However, if the amount of solid residue increases, the task of confirming their internal characteristics becomes extremely troublesome. Namely, in the case of a large amount of solid residue, the difficulty in interpreting images increases and the technical and time burden on the interpreting physician increases. For example, as indicated in FIGS. 2 to 4 in Example 1 to be subsequently described, in the case there are ten or more protrusions of various sizes in only one portion of the transverse colon, since the task of confirming internal characteristics for the purpose of identification must be performed at ten locations or more, the advantages of three-dimensional diagnosis of being able to be performed easily and rapidly end up being lost.

In contrast, in the case residue is absent or in the case only liquid or gel-like residue is present, image interpretation techniques can be used that use three-dimensional images that are able to be interpreted easily and rapidly. Since only polypoid lesions are observed as protrusions in three-dimensional images of the intestine that only contain liquid or gel-like residue, attention is only required to be focused on those portions. In addition, although CT colonography is normally performed in two positions consisting of the supine position and prone position, in cases in which only liquid or gel-like residue is present, the entire large intestine can be observed for the two positions combined. This is because, as a result of residue in a portion of the intestine in which liquid or gel-like residue is concealed when in one position moving to the opposite side as a result of changing body position, the residue can be depicted in the other body position. In this manner, it is important to change to a position that demonstrates fluidity such that solid residue such as solid stool matter is able to be tagged and move within the intestine.

Namely, the liquid preparation for oral administration of the present invention is a liquid preparation for oral administration taken during gastrointestinal imaging by CT colonography that is comprised of an iodine compound, electrolytes, and a water-soluble polymer and/or saline laxative, wherein 300 mL to 1200 mL are taken on the day before CT colonography in a single administration or divided among several administrations.
Pretreatment using the liquid preparation for oral administration of the present invention not only requires a smaller amount of oral colonic irrigation agent than in the prior art, but also succeeds in reducing the number of days required for pretreatment to only one day of the day before the examination, and can be incorporated into hospital testing systems by enabling testing to be performed on a large number of patients both easily and in a short period of time during testing for group screening and general health examinations.

The aspect of taking the liquid preparation for oral administration of the present invention is such that 300 mL to 1200 mL are to be taken on the day before CT colonography, and the 300 mL to 1200 mL may be taken in a single administration or divided among several administrations. For example, 350 mL to 450 mL may be taken by dividing among two to three administrations, or 100 mL to 400 mL may be taken in a single administration or divided among several administrations. In addition, the amount taken in a single administration may be taken all at once or may be taken continuously in small amounts within 30 minutes. Furthermore, the amount of the liquid preparation for oral administration of the present invention taken on the day before CT colonography can be within the range of 300 mL to 1200 mL, and can be determined according to the age, body weight, status and so forth of the subject undergoing CT colonography. In addition, it may be taken after eating, before eating or while eating, and may be taken after waking up, between meals or before going to bed.

For example, by taking 600 mL to 1200 mL of the liquid preparation for oral administration of the present invention on the day before CT colonography in a single administration or divided among several administrations, a state can be obtained in which solid residue is not present in the intestinal tract or only residue in a liquid state or gelatinous state is present in the intestinal tract at the time CT colonography is performed. Namely, intestinal cleansing effects can be obtained that are comparable to the case of taking a large amount on the order of about 2 liters of colonic irrigation solution on the day CT colonography is performed. In the present invention, 350 mL to 450 mL are preferably taken divided among two administrations consisting of once after breakfast and once after dinner.

In addition, the liquid preparation for oral administration of the present invention can also be taken in an amount less than 600 mL, such as 100 mL to 450 mL, in a single administration or divided among two to three administrations on the day before CT colonography. In the case of lowering the total amount taken, it is preferably taken by dividing among two to three administrations rather than taking in a single administration, and is more preferably taken by dividing among two administrations consisting of once after breakfast and once after dinner. In the case the person taking the liquid preparation for oral administration has ordinary bowel movements, even in cases in which the total amount of the liquid preparation for oral administration taken is low at 300 mL to 400 mL, cleansing and tagging of the intestine are carried out in the same manner as in the case of having taken 600 mL to 1200 mL. On the other hand, in cases when smaller amounts are taken depending on the status of bowel movements of the person taking the liquid preparation for oral administration, there are cases in which a comparatively large amount of solid residue may remain in the intestine. In such cases as well, since the liquid preparation for oral administration of the present invention contains an adequate amount of an iodine compound, resulting images can be used for diagnosis and the like as a result of the solid residue in the intestine being able to be adequately tagged.

Since the amount taken of the liquid preparation for oral administration of the present invention is sufficiently small in comparison with the ordinary amount (2 liters) of oral colonic irrigation agent used in conventional colon examinations, it can be taken in a short period of time, thereby placing little burden on the subject. Since this burden is reduced particularly for elderly persons who have difficulty in drinking a large amount of liquid all at once, the liquid preparation for oral administration of the present invention is preferable as an examination drug for elderly persons. In addition, it is also preferable as an examination drug for group screening targeted at a wide range of age groups including the elderly.

The contrast media used in CT colonography is generally divided into barium such as barium sulfate and iodine-based contrast media. Barium has difficulty in uniformly mixing with residue in the intestine and since it precipitates due to a difference in specific gravity, it has the problem of forming a layer even during imaging. In addition, due to its high CT value, there is the possibility of lesions being concealed due to the occurrence of pseudoenhancement, while also having the problem of preventing CT colonography and endoscopy from being performed on the same day. Since the liquid preparation for oral administration of the present invention uses an iodine-based contrast medium instead of barium, in addition to being able to obtain highly reliable imaging results by CT colonography, it also enables CT colonography and endoscopy to be performed on the same day.

There are no particular limitations on the iodine compound contained in the liquid preparation for oral administration of the present invention provided it has one or more iodine atoms in a molecule thereof and is a water-soluble organic compound that can be used as a contrast medium. Examples of such water-soluble organic iodine compounds include those having a triiodobenzenes in which iodine is bonded at positions 2, 4 and 6 of a benzene ring. Although the iodine compound contained in the liquid preparation for oral administration of the present invention may be an ionic compound or a nonionic compound, it is preferably a nonionic compound. This is because nonionic iodine compounds taste better, are taken more easily and cause little concern over allergic reactions in comparison with ionic iodine compounds.

Specific examples of ionic iodine compounds include amidotrizoic acid (chemical name: 3,5-diacetamino-2,4,6- triiodobenzoic acid), meglumine sodium amidotrizoate, meglumine amidotrizoate, sodium iothalamate, meglumine iothalamate, meglumine iotroxate, iotrolan, ioxaglic acid, ioxilan and iopromide. In addition, specific examples of nonionic iodine compounds include iopamidol, iohexol and iomeprol.

Each of these iodine compounds may have adverse side effects such as an anaphylactic reaction, and can be suitably selected according to patient background such as whether or not the patient has an allergic constitution. In the case of using an ionic iodine compound, the use of sodium meglumine amidotrizoate is preferable particularly in patients for which there is little risk of an allergic reaction or other adverse side effects in terms of its pharmaceutical stability and cost.

As is also described in Patent Document 2, it is necessary to perform electronic cleansing in order to accurately reconfigure two-dimensional images obtained by CT imaging to three-dimensional images. In order to perform electronic cleansing efficiently, the CT values of portions where liquid residue has accumulated must be sufficiently high. In addition, in the case of performing CT colonography as a primary screening test for group screening and general health examinations and the like, although it is preferable that imaging be performed at a low exposure dose of 10 mSv or less, in cases where there is a small difference in CT values between residue and lesions, lowering the dose during imaging makes it difficult to distinguish between polyp lesions and residue tagged with oral contrast media. Consequently, it is necessary to make the CT value of residue sufficiently higher than that of polyp lesions. During CT colonography in the case of carrying out pretreatment using the liquid preparation for oral administration of the present invention, the CT value of residue is preferably 300 HU or more, and is preferably uniformly maintained within the range of 300 HU to 700 HU at each site in the intestine. As a result of residue CT values being within this range, residue and polyp lesions (having CT values of less than 100 HU) can be adequately distinguished even if imaging at a low exposure dose.

CT values of portions where liquid residue has accumulated can be adjusted according to the iodine content and amount taken of the liquid preparation for oral administration of the present invention taken on the day before CT colonography. Consequently, the iodine content of the liquid preparation for oral administration of the present invention can be suitably adjusted in consideration of the amount taken, CT values of residue during CT colonography after taking, the status of the subject and the like.

The iodine content of the liquid preparation for oral administration of the present invention is preferably 90 mg/mL or less, more preferably 70 mg/mL or less, even more preferably 35 mg/mL or less, and particularly preferably 28 mg/mL or less. In addition, the iodine content of the liquid preparation for oral administration of the present invention is preferably 3.5 mg/mL or more, more preferably 7 mg/mL or more, even more preferably 10 mg/mL or more, and particularly preferably 15 mg/mL or more.

The liquid preparation for oral administration of the present invention also contains at least one of a water-soluble polymer and saline laxative in addition to an iodine compound. In the present invention, an iodine compound and a water-soluble polymer are preferably contained in order to further reduce the burden on the subject. Furthermore, the liquid preparation for oral administration of the present invention may also contain both a water-soluble polymer and a saline laxative.

Examples of water-soluble polymers contained in the liquid preparation for oral administration of the present invention include polyethylene glycol, polydextrose, dextran, dextrin, hydroxyethyl starch, gum arabic, pullulan, pectin, and carboxymethyl cellulose. Among these, a water-soluble polymer selected from the group consisting of polyethylene glycol, polydextrose, dextran, hydroxyethyl starch, gum arabic, pullulan and pectin is preferable in terms of pharmaceutical stability and the like.

The liquid preparation for oral administration of the present invention particularly preferably contains polyethylene glycol for the water-soluble polymer. The molecular weight of polyethylene glycol used is preferably 2000 to 8000 and more preferably 3000 to 7000.

Examples of saline laxatives contained in the liquid preparation for oral administration of the present invention include magnesium citrate, sodium dihydrogen phosphate and disodium hydrogen phosphate. There are no particular limitations on the concentration of saline laxative contained in the liquid preparation for oral administration of the present invention provided it is of a concentration capable of demonstrating laxative action, and can be suitably adjusted in consideration of the type of saline laxative and the status and so forth of the patient taking it. For example, a sodium ion concentration of 60 mEq/L, potassium ion concentration of 20 mEq/L, magnesium ion concentration of 3 mEq/L, chloride ion concentration of 50 Eq/L, phosphate ion concentration of 10 mmol/L, citrate ion concentration of 35 mEq/L and lactate ion concentration of 20 mEq/L do not have an excessive effect on electrolyte balance in the body. Consequently, the saline laxative is added so that the content in the liquid preparation for oral administration of the present invention is higher than these concentrations.

During pretreatment using the liquid preparation for oral administration of the present invention, solid residue is to be effectively removed from the intestine of the subject, although liquid or gelatinous contents may remain. Namely, since the objective of the liquid preparation for oral administration of the present invention is not to completely cleanse and remove contents such as solid stool matter from the intestine or cleanse and remove the majority thereof, the burden on the subject is more greatly reduced than conventional pretreatment using colonic irrigation.

Osmotic pressure of the liquid preparation for oral administration of the present invention is preferably 200 mOsm/L to 440 mOsm/L. As a result of osmotic pressure being within this range, fluctuations in the balance of serum electrolytes in the body and osmotic pressure resulting from taking the liquid preparation for oral administration can be minimized. The liquid preparation for oral administration of the present invention is more preferably adjusted to an osmotic pressure range of 280 mOsm/L to 320 Osm/L or to an osmotic pressure that is nearly isotonic.

The liquid preparation for oral administration of the present invention may also be a preparation such that osmotic pressure in the intestine is adjusted by combining with drinking water and the like in the manner of the colonic irrigation solution described in, for example, Japanese Patent Publication No. 4131266. In the case of taking by combining with drinking water and the like, the osmotic pressure of the liquid preparation for oral administration of the present invention may be higher than the aforementioned osmotic pressure range, such as an osmotic pressure of 700 mOsm/L or less.

The liquid preparation for oral administration of the present invention preferably contains electrolytes in order to adjust to a desired osmotic pressure. The types and incorporated amounts of electrolytes contained in the liquid preparation for oral administration of the present invention are suitably selected so that osmotic pressure of the liquid preparation for oral administration is within the aforementioned range.

Electrolytes refer to substances that form ions by dissociating in solution, and examples thereof include Na⁺, K⁺, Ca²⁺, Mg²⁺, HCO₃⁻, SO₄²⁻, HPO₄²⁻, organic acid groups and organic base groups. For example, electrolytes are also contained in the saline laxative. Examples of electrolytes contained in the liquid preparation for oral administration of the present invention include the same electrolytes as those used for intravenously administered electrolyte infusions and the like. More specifically, examples of sodium ion sources include sodium chloride, sodium acetate, sodium citrate, sodium dihydrogen phosphate, disodium hydrogen phosphate, sodium sulfate and sodium lactate, examples of potassium ion sources include potassium chloride, potassium acetate, potassium citrate, potassium dihydrogen phosphate, dipotassium hydrogen phosphate, potassium sulfate and potassium lactate, examples of calcium ion sources include calcium chloride, calcium gluconate, calcium pantothenate, calcium lactate, calcium acetate and calcium glycerophosphate, examples of magnesium ion sources include magnesium sulfate, magnesium chloride, magnesium acetate and magnesium citrate, examples of phosphate ion sources include sodium dihydrogen phosphate, disodium hydrogen phosphate and sodium glycerophosphate, examples of chloride ion sources include sodium chloride, potassium chloride, calcium chloride and magnesium chloride, and examples of carbonate ion sources include sodium bicarbonate, and these compounds may also be in the form of hydrates.

The liquid preparation for oral administration of the present invention may further contain a sugar. A mixture of electrolytes and sugar is particularly preferably added to a liquid preparation for oral administration preferable for pretreatment of CT colonography performed as a screening test for reasons of pharmaceutical stability and superior patient acceptance, including ease of taking.

The sugar contained in the liquid preparation for oral administration of the present invention may be a sugar or sugar-alcohol. Specific examples of sugars contained in the liquid preparation for oral administration of the present invention include sucrose, sorbitol, xylitol, erythritol, mannitol, trehalose, lactitol, lactulose, maltitol, palatinose, raffinose and glycerin. Among these, xylitol, sorbitol, lactulose, lactitol and raffinose are particularly preferable for reasons of pharmaceutical stability and patient acceptance including ease of taking and the like.

The total incorporated amount of water-soluble polymer and electrolytes in the liquid preparation for oral administration of the present invention, or the total incorporated amount of water-soluble polymer, electrolytes and sugar is, for example, 2 g to 40 g, and preferably 10 g to 30 g, in 400 mL of the liquid preparation. In order to maintain serum electrolyte balance, in which the amount of electrolytes absorbed from the intestine is offset by the amount of electrolytes secreted into the intestine, electrolytes are preferably added so that the liquid preparation for oral administration of the present invention has an Na⁺ concentration of 30 mEq/L to 150 Eq/L, K⁺ concentration of 3 mEq/L to 20 mEq/L, Cl⁻ concentration of 20 mEq/L to 70 mEq/L and HCO₃⁻ concentration of 10 mEq/L to 50 mEq/L. In addition to these electrolytes, poorly absorbed ions in the manner of magnesium ions and sulfate ions are preferably added to the liquid preparation for oral administration in order to inhibit absorption of water and electrolytes in the intestine. In the case of incorporating magnesium ions, the concentration of magnesium ions in the liquid preparation for oral administration is preferably adjusted to 40 mEq/L to 120 mEq/L. In the case of incorporating sulfate ions, the concentration of sulfate ions in the liquid preparation for oral administration is preferably adjusted to 40 mEq/L to 120 mEq/L. In the case of further using a sugar in addition to electrolytes and water-soluble polymer, the amount of sugar used is 2 g to 40 g, and preferably 5 g to 10g, in the liquid preparation for oral administration.

In addition to an iodine compound, electrolytes, water-soluble polymer and saline laxative, the liquid preparation for oral administration of the present invention preferably contains a gastrointestinal function accelerator. The use of a gastrointestinal function accelerator enables the ingested liquid preparation for oral administration or mixture of liquid preparation for oral administration and intestine contents to be easily excreted, thereby making it possible to decrease the amount of intestinal residue during CT colonography. In particular, the liquid preparation for oral administration of the present invention preferably further contains a gastrointestinal function accelerator in the case the total amount taken on the day before CT colonography is comparatively small.

An example of the gastrointestinal function accelerator contained in the liquid preparation for oral administration of the present invention is a sertonin 5-HT₄ receptor agonist. Examples of sertonin 5-HT₄ receptor agonists include benzoamide derivatives represented by the general formula described in claim 1 of the scope of claim for patent of Japanese Examined Patent Application, Second Publication No. H3-54937 including mosapride citrate, as well as cispride and metoclopramide. Among these compounds, mosapride citrate is most preferable from the viewpoint of having few adverse side effects such as induction of arrhythmia ("Mosapride and Enterokinesis", Medical Review Co., Ltd., 1998). Mosapride citrate is preferably contained in the liquid preparation for oral administration of the present invention after suitably adjusting so that the amount taken per day (namely, the amount used in pretreatment for performing one round of CT colonography) is 5 mg to 40 mg.

Commonly used large intestine stimulant laxatives can also be contained as a gastrointestinal function accelerator in the liquid preparation for oral administration of the present invention. Examples of large intestine stimulant laxatives include sodium picosulfate, bisacodyl and sennoside. Large intestine stimulant laxatives can be contained in the liquid preparation for oral administration of the present invention by suitably adjusting so that the amount used in pretreatment for one round of CT colonography is the prescribed daily dose of that preparation, or the prescribed amount taken for pretreatment of large intestine examinations. For example, sodium picosulfate is preferably contained in the liquid preparation for oral administration of the present invention by suitably adjusting so that the amount taken per day (namely, the amount used in pretreatment for performing one round of CT colonography) is 5 mg to 150 mg.

In general, difficulty in detecting lesions in images containing intestinal foamy mucous is frequently encountered in CT colonography examinations. There are also cases in which it may be difficult to detect lesions in images in which intestinal foamy mucous is present even if performing pretreatment by taking the liquid preparation for oral administration of the present invention. Consequently, the liquid preparation for oral administration of the present invention preferably also contains dimethylpolysiloxane in addition to an iodine compound, water-soluble polymer and saline laxative. Since dimethylpolysiloxane demonstrates antifoaming action on bubbles present in foamy mucous, the accuracy and efficiency of detecting lesions in captured images can be improved.

An example of the dimethylpolysiloxane contained in the liquid preparation for oral administration of the present invention is "Gascon™" available commercially as a gastrointestinal gas eliminator. The dimethylpolysiloxane contained in the liquid preparation for oral administration of the present invention is preferably contained therein by suitably adjusting so that the daily adult dosage (namely, the amount used in pretreatment for performing one round of CT colonography) is 30 mg to 120 mg and preferably 40 mg to 80 mg. Furthermore, the liquid preparation for oral administration of the present invention not containing dimethylpolysiloxane and a liquid preparation containing dimethylpolysiloxane may also be separately taken orally on the day before CT colonography either in a single administration or by dividing among several administrations.

Furthermore, although dimethylpolysiloxane has conventionally been used for improvement of abdominal symptoms attributable to gas in the gastrointestinal tract, elimination of foamy mucous in the stomach when performing gastroscopy, and elimination of intestinal gas during abdominal X-ray examinations and the like, it had not been previously used for pretreatment of CT colonography. Moreover, since the antifoaming action of dimethylpolysiloxane differs from the conventional role of dimethylpolysiloxane, the improvements in accuracy and efficiency of detecting lesions in captured images was an unexpected finding.

There are many cases in which iodide compounds, water-soluble polymers and electrolytes have a characteristic taste and smell. Therefore, the liquid preparation for oral administration of the present invention is preferably provided with means for correcting taste and smell. More specifically, the liquid preparation for oral administration of the present invention can incorporate a sweetener or fragrance.

There are no particular limitations on the fragrance contained in the liquid preparation for oral administration of the present invention provided it masks odors attributable to the water-soluble polymer and electrolytes when orally ingesting the liquid preparation for oral administration. In the present invention, food fragrances are suitable, and fruit fragrances are particularly suitable. Examples of fruit fragrances include lemon, orange, grapefruit, lemon-lime, mandarin orange, grape, strawberry, cherry, apple, apricot and raspberry fragrances. Among these, citrus-based liquid fragrances in the manner of lemon, orange, grapefruit and lemon-lime are optimal since they impart a refreshing sensation during drinking. Refined oils obtained by squeezing from fruits or by steam distillation can be used as liquid fragrances. In addition, substances such as limonene, citral, citronellal, linalool or octanal can be used alone or blended. Among these, citrus-based fragrances containing limonene are used preferably. These fragrances are commercially available from fragrance manufacturers, and it is realistic to use such fragrances. Furthermore, liquid fragrances include water-soluble liquid fragrances obtained by extracting or dissolving fragrance components with aqueous alcohol and the like, and oil-soluble liquid fragrances obtained by dissolving fragrance components in an oily solvent. Moreover, fragrances in which a preservative and the like have been added to the fragrance component (refined oil) are also included. Oily fragrances or fragrances derived from refined oil are used preferably as liquid fragrance instead of water-soluble liquid fragrances from the viewpoint of storage stability.

In the case of using a fragrance, it is necessary to adjust the amount used in consideration of vaporization in each production step, adhesion to the package as well as permeation and dissipation from the package. The preferable added amount is 0.001% by weight to 0.3% by weight of the liquid preparation for oral administration. In the present invention, the fragrance is directly adsorbed to an electrolyte powder. Incorporation of fragrance by preliminarily adsorbing to a sugar must be avoided since there is the risk of generation of hydrogen gas and methane gas.

The sweetener used in the present invention is used for the purpose of making it easier to drink the liquid preparation for oral administration when taking in large amounts. The sweetener must be that which does not generate hydrogen gas and methane gas, or only generates extremely small amounts thereof, in the intestine. However, in cases when not using a procedure for electrically removing a polyp simultaneous to endoscopy (endoscopic polypectomy) by performing endoscopy following CT colonographic imaging, a sugar, and particularly sucrose (refined white sugar) may be aggressively added to improve ease of drinking the liquid preparation and to replenish energy. Specific examples of preferable sweeteners that produce no or only extremely small amounts of hydrogen gas and methane gas in the intestine include saccharine, sodium saccharine, acesulfame-K, cyclamate (sodium cyclohexylsulfamate) and aspartame, and these can be used either alone or in combination. The added amount of these sweeteners is preferably 0.001 % by weight to 0.3% by weight, and more preferably 0.01% by weight to 0.3% by weight, of the liquid preparation for oral administration.

The liquid preparation for oral administration of the present invention can also contain other components provided they do not inhibit the effects of the present invention. For example, the liquid preparation for oral administration of the present invention may contain ascorbic acid and/or one or more types of a salt thereof (ascorbate component). For example, this ascorbate component is preferably added within the range of 4 g to 15 g per liter of the liquid preparation for oral administration of the present invention. In the case the total amount taken on the day before CT colonography is a comparatively small amount in particular, the liquid preparation for oral administration of the present invention more preferably contains an ascorbate component.

A preferable salt of ascorbic acid is an alkaline metal salt or alkaline earth metal salt, examples of which include sodium ascorbate, potassium ascorbate, magnesium ascorbate and calcium ascorbate. A particularly preferable salt of ascorbic acid is sodium ascorbate. The ascorbate component preferably contains both ascorbic acid and one or more types of a salt thereof. The ascorbic acid and salt are preferably present at a weight ratio within the range of 1:9 to 9:1. Ascorbic acid and salt thereof can actually be provided as a hydrate. If a hydrate is used, the previously described weights and/or weight ratios refer to the weights and/or weight ratios of the ascorbic acid or salt excluding the hydration water. The weight ratio of ascorbic acid and salt thereof is preferably within the range of 2:8 to 8:2, more preferably 3:7 to 7:3, and even more preferably 4:6 to 6:4, such as a weight ratio of 4.7 to 5.9.

As a result of using the liquid preparation for oral administration of the present invention in pretreatment for CT colonography, an iodine compound can be contained that is favorable for liquid or gelatinous residue present in deep portions of the large intestine. Consequently, pretreatment using the liquid preparation for oral administration of the present invention is particularly useful for examinations of sites in deeper portions of the large intestine such as the colon or rectum.

The liquid preparation for oral administration of the present invention may also be that obtained by adding a contrast medium having for an active ingredient thereof an iodine compound to an oral colonic irrigation agent (colonic irrigation solution) used when performing colonic irrigation as pretreatment for endoscopic colonoscopy, barium enema and CT colonography. Namely, that obtained by combining an iodine-based water-soluble contrast medium with an oral colonic irrigation agent followed by adjusting the iodine content to 3.5 mg/mL to 90 mg/mL can be used for the liquid preparation for oral administration of the present invention.

Although there are no particular limitations on this type of oral colonic irrigation agent provided it contains at least one of water-soluble polymer and saline laxative, it preferably also contains electrolytes so that the osmotic pressure when taken is 200 mOsm/L to 440 mOsm/L, and more preferably contains a sweetener and/or fragrance. Specific examples thereof include that composed of a composition consisting of the combination of polyethylene glycol and electrolytes (for example, "Niflec™" internal preparation, Ajinomoto Pharmaceuticals Co., Ltd.) according to an invention disclosed in Japanese Unexamined Patent Application, First Publication No. H1-125319), that composed of a composition consisting of the combination of electrolytes and at least one type of water-soluble polymer selected from the group consisting of dextran, dextrin, hydroxyethyl starch, polydextrose, gum arabic and pectin (Japanese Unexamined Patent Application, First Publication No. H2-25424, Japanese Unexamined Patent Application, First Publication No. H3-206046), that composed of a composition consisting of the combination of erythritol or xylitol and electrolytes (Japanese Unexamined Patent Application, First Publication No. H3-284620), that composed of a composition consisting of the combination of fructooligosaccharides and electrolytes (Japanese Unexamined Patent Application, First Publication No. H3-291228), a composition containing electrolytes and at least one type of substance selected from the group consisting of lactitol, maltitol and carboxymethyl cellulose (Japanese Unexamined Patent Application, First Publication No. H5-255092), magnesium citrate (Japanese Unexamined Patent Application, First Publication No. H5-306221), and an isotonic solution obtained by using a tonicity agent and a saline laxative such as sodium dihydrogen phosphate or disodium hydrogen phosphate.

The liquid preparation for oral administration of the present invention can be prepared by preparing a concentrate containing each of the components and diluting with a suitable amount of water at the time it is taken. The concentrate of each component may be in a form that is filled into an aluminum can, plastic container or hard or semi-hard container. At this time, the solution is naturally disinfected or sterilized in accordance with normal methods (see, for example, Japanese Unexamined Patent Application, First Publication No. H9-58747 or Japanese Unexamined Patent Application, First Publication No.H8-253220).

In addition, the liquid preparation for oral administration of the present invention can also be prepared by preparing each component that has been respectively formed into a powder, tablets or granules, and dissolving in a suitable amount of water at the time it is taken. A composition may also be prepared in which two or more types of components are mixed in advance, followed by dissolving the composition and other components in a suitable amount of water when taken.

For example, the liquid preparation for oral administration of the present invention can be prepared by dissolving both a powdered preparation, which contains at least one of a water-soluble polymer and a saline laxative and electrolytes other than those of the saline laxative as necessary and allows the preparation of a colonic irrigation solution having osmotic pressure of 200 mOsm/L to 440 mOsm/L by dissolving in a prescribed amount of water (such as "Niflec™" internal preparation, Ajinomoto Pharmaceuticals Co., Ltd.) according to an invention disclosed in Japanese Unexamined Patent Application, First Publication No. H1-125319), and a contrast medium having an iodine compound as an active ingredient thereof, in a prescribed amount of water when taken. Furthermore, that formulated into the form of tablets or granules may also be used instead of a powdered preparation.

In addition, a composition containing at least one of a water-soluble polymer and a saline laxative and an iodine compound (and as necessary, electrolytes other than those of the saline laxative, dimethylpolysiloxane and gastrointestinal function accelerator) may be prepared in advance, followed by dissolving the composition with other components in the form of a sweetener and fragrance in a suitable amount of water when taken. A liquid preparation for oral administration having a desired taste and smell can be more easily prepared by preliminarily preparing active ingredients consisting of an oral colonic irrigation agent and contrast medium in the form of a single composition, and separately preparing components having a corrective action in the manner of a sweetener or fragrance.

Among the components that compose the liquid preparation for oral administration of the present invention, the composition for gastrointestinal radiography of the present invention refers to a composition that at least contains an iodine compound and a water-soluble polymer or saline laxative. Namely, the composition for gastrointestinal radiography of the present invention comprises the preparation of an aqueous solution that contains an iodine compound and at least one of a water-soluble polymer and saline laxative, and in which the iodine content is 3.5 mg/mL to 90 mg/mL, by dissolving in or diluting with water. Furthermore, a composition containing components such as electrolytes other than those of the saline laxative, dimethylpolysiloxane, gastrointestinal function accelerator, sweetener or fragrance is also included in the composition for gastrointestinal radiography of the present invention. In addition, the osmotic pressure of an aqueous solution obtained by dissolving or diluting the composition for gastrointestinal radiography of the present invention in water is preferably 200 mOsm/L to 440 mOsm/L.

The composition for gastrointestinal radiography of the present invention is preferably obtained by mixing components that compose the liquid preparation for oral administration of the present invention, excluding the iodine compound, after having adjusted the particle diameter so as to enable each component to be mixed.

A composition containing all components that compose the liquid preparation for oral administration of the present invention including the iodine compound is preferably distributed by filling into an airtight container and placing in a sealed package. In order to ensure ease of taking and convenience during use in particular, the composition is preferably prepared so that the daily amount taken, or the amount taken during a single administration in the case of dividing among multiple administration (such as the amount used by dissolving in 350 mL to 450 mL), is the amount contained in a single package.

The composition for gastrointestinal radiography of the present invention is filled into a package in the manner of an aluminum or other metal pouch or can lined with a thermoplastic resin, or a plastic package formed with a thermoplastic resin. In this case, since a fragrance containing limonene is easily absorbed by polyolefin-based thermoplastic resins in particular or is easily dissipated as a result of permeating there through, it is necessary to employ a package such that an adequate amount of fragrance remains at the time of use. Since the inner surface area of the package as well as the thickness of the resin layer influence the adsorbed amount and the amount that is dissipated by permeation, it is necessary to set the thickness of the resin layer and the like within a range that does not lead to a decrease in package sealing strength and allows an adequate amount of fragrance to remain at the time of use. A package in which the adsorbed amount of limonene or the amount of limonene that dissipates due to permeation at the time of use is 1000 mg or less per package is particularly preferable.

The composition for gastrointestinal radiography of the present invention can be taken by transferring from the package to a container and dissolving with water. However, using a plastic container capable of holding the required amount of dissolving water (for example, 200 mL to 1200 mL) for the package per se, and placing dissolving water directly in the package at the time of use to dissolve prior to taking provides convenience since it is not necessary to prepare a separate container for dissolving. In addition, a scale allowing the amount taken per administration when taking by dividing among several administrations to be determined visually is preferably indicated on the package.

The plastic container in this case is preferably formed with a polyolefin-based thermoplastic resin. Examples of polyolefin-based thermoplastic resins include polyethylene and polypropylene. Although soft polyolefins result in favorable adhesion between resins that is required in terms of molding, these materials also have high levels of gas adsorption and permeability with respect to fragrances containing limonene. On the other hand, although hard polyolefins have low levels of gas adsorption and permeability, there is poor adhesion between resins. Thus, in order to satisfy both molding requirements and gas adsorption and permeability requirements, if a soft polyolefin is used for the inner layer (surface that contacts the composition for gastrointestinal radiography) and a hard polyolefin is layered thereon, a large-capacity package can be obtained that has low overall gas adsorption and permeability, demonstrates favorable adhesion (moldability), and can also be used as a container for dissolving. Moreover, a package having a total of three layers, obtained by layering a soft polyolefin for the innermost layer (surface that contacts the composition for gastrointestinal radiography), a hard polyolefin on the outside thereof, and a soft polyolefin on the outside of the hard polyolefin, is particularly preferable.

A soft polyolefin, and particularly a linear polyethylene, is preferably used for the innermost layer. The innermost layer in this case is preferably formed with a polyolefin-based thermoplastic resin in which the thickness of the innermost layer is 100 µm or less and preferably 50 µm or less in order to inhibit reductions in the fragrance containing limonene due to adsorption or dissipation caused by permeation. In the case of forming a plastic package having two layers consisting of an inner layer composed of a soft polyolefin and an outer layer composed of a hard polyolefin, the total thickness is preferably 70 µm to 200 µm, and the respective thicknesses of the inner layer and outer layer are preferably 35 µm to 100 µm. In the case of forming a plastic package composed of three layers by layering a soft polyolefin for the innermost layer, a hard polyolefin on the outside thereof, and a soft polyolefin on the outside of the hard polyolefin, the total thickness is preferably 70 µm to 200 µm, and the respective thicknesses of each layer are preferably 15 µm to 70 µm and particularly preferably 20 µm to 40 µm.

The gas permeability of the package is preferably 20 cc/m² · day · atm (25°C) or less in order to achieve both objectives of inhibiting decreases in the content of sodium bicarbonate among the active ingredients caused by generation of carbon dioxide gas over time and preventing dissipation of limonene from the package.

A package having enhanced strength with respect to impacts caused by dropping or puncturing of the package is preferably formed by further laminating a thermoplastic resin such as polyethylene terephthalate or polyamide on the outside of the polyethylene layer of the aforementioned second or third layer.

Although there are no particular limitations thereon, the form of the package is preferably provided with a dissolving water filler hole in the case the package is also used as a plastic container capable of holding dissolving water, and preferably provided with a bottom surface that allows it to be stood up after being used to dissolve components. When considering ease of production, the package preferably has two nearly triangular side walls of a height higher than the height of the base, a filler hole in the upper portion of the side walls, and a bottom having an oblong shape attached to the lower portions of the side walls. The two side walls and bottom are composed of a flexible plastic, the filler hole is composed of a hard plastic, and a cover is attached to the filler hole. By employing a structure in which the two sidewalls and bottom are composed of a flexible plastic and the bottom is folded to the inside until dissolving water is added, the entire package can be made to be compact until dissolving water is added if air is expelled from inside the package (container), thereby making this favorable for storage and transport. The two side walls are preferably composed of transparent or semi-transparent plastic so as to allow the contained amount to be confirmed visually. The amount taken can be visually confirmed when taking without requiring the use of a measuring cup to add dissolving water by providing a scale indicating the amount of dissolving water on the side walls.

The composition for gastrointestinal radiography of the present invention can be prepared by mixing the prescribed components using a conventionally known method. In the case of filling into a package, removing as much air as possible makes it possible to prevent deterioration of the quality of active ingredients that are unstable in the presence of oxygen over time, while also providing convenience during storage and transport since the package is not bulky. In addition, in the case of requiring long-term storage, the package can be placed and sealed in a gas-impermeable packaging pouch formed with an aluminum laminated film and the like.

Each component and composition used to prepare the liquid preparation for oral administration of the present invention, including the composition for gastrointestinal radiography of the present invention, is preferably in the form of a single kit or packaged product. For example, a combination of the aforementioned oral colonic irrigation agent and iodine-containing contrast agent, a combination of a respectively and individually prepared iodine compound and water-soluble polymer that enables them to be blended, a combination of a respectively and individually prepared iodine compound and saline laxative that enables them to be blended, or a combination of a respectively and individually prepared iodine compound, water-soluble polymer and saline laxative that enables them to be blended, for example, can be used as pretreatment pharmaceuticals for CT colonography.

There are no particular limitations on the fabrication of such a kit or packaged product, and they can be suitably fabricated according to normal methods. Namely, at least each of the components and compositions for preparing the liquid preparation for oral administration of the present invention are contained in a single package form. The kit and packaged product preferably contain instructions describing the order in which components are taken and instructions for use, symptoms for which administration must be discontinued, and warnings describing actions to be taken in the event of adverse side effects. Moreover, they can also contain a cup, a check sheet used when taking, a timer, dissolving liquid and the like.

As an example thereof, a powdered preparation, which contains electrolytes and a water-soluble polymer (and as necessary, a sugar, dimethylpolysiloxane and gastrointestinal function accelerator) and allows the preparation of a colonic irrigation solution having osmotic pressure of 200 mOsm/L to 440 mOsm/L by dissolving in a prescribed amount of water, is filled into a plastic container (see Japanese Unexamined Patent Application, First Publication No. H4-259461), and a blister pack, which separately houses a solution or tablet containing an iodine compound along with instructions describing the order in which components are taken and warnings describing symptoms of adverse side effects and actions to be taken in the event of their occurrence, is removably attached thereto. A form in which the aforementioned plastic container having a blister pack removably attached thereto is inserted into or attached to the outer wall of a container for dissolving the composition for gastrointestinal radiography is useful. Convenience is further enhanced by preliminarily filling a dissolving liquid such as mineral water into the aforementioned container for dissolving.

In addition, the aforementioned blister pack may also be attached to a container filled with a colonic irrigation solution that contains electrolytes and a water-soluble polymer (and as necessary, a sugar, dimethylpolysiloxane and gastrointestinal function accelerator) and has osmotic pressure of 200 mOsm/L to 440 mOsm/L.

As another example thereof, a favorable example of a package form is a bag for housing a kit referred to as a triple bag, in which each component and composition of the liquid preparation for oral administration of the present invention is able to be contained in three compartments obtained by dividing a flexible plastic bag by fusing portions of the bag. This kit bag enables a liquid preparation for oral administration to be prepared by separating the intermediate fused portions by pressing by hand at the time of use, and mixing each of the powdered components and compositions with the dissolving liquid. Printing instructions and warnings on the surface of this kit bag enables a single bag to be formed into a kit (Design Registration No. 2000-2619).

Since contrast media irritates inflammation of intestinal mucosa in patients with inflammatory bowel disease resulting in the risk of having a detrimental effect on ulcerations and other affected areas, a steroid may also be contained in the kit or packaged product for the purpose of preventing inflammation.

### [Examples]

Although the following provides a more detailed explanation of the present invention by indicating examples thereof, the present invention is not limited to the following examples.

### [Example 1]

Images of the large intestine obtained by CT imaging were evaluated for the case of taking 800 mL of the liquid preparation for oral administration of the present on the day before CT colonography (Group A, 2 subjects), the case of taking 800 mL of a solution containing an iodine compound but not containing a water-soluble polymer or saline laxative (not containing electrolytes at a concentration sufficient for demonstrating a laxative action) (Group B, 2 subjects), the case of taking 400 mL (half the amount of Group A) of the same liquid preparation as Group A (Group C, 3 subjects), and the case of taking 40 mg of mosapride citrate in addition to taking the same liquid preparation as Group A in an equal amount as Group A (Group D, 1 subject).

Specific pretreatment of each group was as indicated below. Furthermore, Omnipaque™ Injection 350 (trade name, Daiichi Sankyo Co., Ltd., generic name: Iohexol), Ioverin™ Injection 350 (trade name, Taiyo Pharmaceutical Co., Ltd., generic name: Iohexol), or Optiray™ 350 (trade name, Covidien Japan Inc., generic name: Ioversol) was used for the iodine-based water-soluble contrast medium.

Group A: A total of 400 mL of a liquid preparation obtained by incorporating 20 mL of an iodine-based water-soluble contrast medium in 380 mL of an oral colonic irrigation agent (Niflec™ internal preparation, trade name, Ajinomoto Pharmaceuticals Co., Ltd.) were taken internally at 7:00 AM on the day before the examination, and 400 mL of the same liquid preparation were taken internally at 7:00 PM on the day before the examination. The total amount taken in one day was 800 mL (consisting of 760 mL of oral colonic irrigation agent and 40 mL of iodine-based water-soluble contrast medium).

Group B: A total of 400 mL of a liquid preparation obtained by mixing 20 mL of an iodine-based water-soluble contrast medium with 380 mL of a sports drink (Pocari Sweat, trade name, Otsuka Pharmaceutical Co., Ltd.) were taken internally at 7:00 AM on the day before the examination, and 400 mL of the same liquid preparation were taken internally at 7:00 PM on the day before the examination. The total amount taken in one day was 800 mL (consisting of 760 mL of sports drink and 40 mL of iodine-based water-soluble contrast medium).

Group C: A total of 400 mL of a liquid preparation obtained by incorporating 20 mL of an iodine-based water-soluble contrast medium in 380 mL of an oral colonic irrigation agent (Niflec™ internal preparation, trade name, Ajinomoto Pharmaceuticals Co., Ltd.) were taken internally at 9:00 PM before bed on the day before the examination. The total amount taken in one day was 400 mL (consisting of 380 mL of oral colonic irrigation agent and 20 mL of iodine-based water-soluble contrast medium).

Group D: A total of 400 mL of a liquid preparation obtained by incorporating 20 mL of an iodine-based water-soluble contrast medium in 380 mL of an oral colonic irrigation agent (Niflec™ internal preparation, trade name, Ajinomoto Pharmaceuticals Co., Ltd.) were taken internally at 7:00 AM on the day before the examination, followed by taking 20 mg of mosapride citrate (Gasmotin™, trade name, Dainippon Sumitomo Pharma Co., Ltd.) with a small amount of water. Later at 7:00 PM on the day before the examination, 400 mL of the same liquid preparation were taken internally followed by taking 20 mg of mosapride citrate with a small amount of water. The total amount taken in one day was 800 mL (consisting of 760 mL of oral colonic irrigation agent and 40 mL of iodine-based water-soluble contrast medium).

MDCT imaging was performed at 9:00 AM on the day after having taken the oral colonic irrigation agent or sports drink containing the iodine-based water-soluble contrast medium. A multi-slice CT system (Toshiba Medical Systems Corp.) was used for MDCT. Three-dimensional image processing was carried out using Colon Analysis Software (AZE Ltd.) capable of three-dimensional image processing, and two types of three-dimensional images (endoscope-like images and enema-like images combining images of liquid residue and gas) were constructed from image data captured by transrectal injection of carbon dioxide gas. In addition, two-dimensional images in the form of axial images were constructed, and the degree of residue tagging by the contrast medium and the physical state of the residue were compared among the three groups of Groups A to C, followed by an evaluation of the quality of intestinal pretreatment using each of the methods. Residue was evaluated by dividing the large colon into six sections consisting of the cecum (C), ascending colon (A), transverse colon (T), descending colon (D), sigmoid colon (S) and rectum (R).

Table 1 indicates the results of evaluating the degree of residue tagging in the intestine by the iodine-based water-soluble contrast medium, while Table 2 indicates the results of evaluating the state of residue in the intestine. In Table 1, "good" indicates that the contrast medium was evenly mixed into the residue, "moderate" indicates that the contrast medium was unevenly mixed into the residue, and "poor" indicates that only a small amount or none of the contrast medium was contained in the residue. In addition, the "--" symbols in Tables 1 and 2 indicate that an evaluation was not performed due to the absence of residue.

**[Table 1]**

| Colon segment | Group A | | Group B | | Group C | | | Group D |
|---|---|---|---|---|---|---|---|---|
| | Case 1 | Case 2 | Case 3 | Case 4 | Case 5 | Case 6 | Case 7 | Case 8 |
| R | Good | Good | Good | Moderate | Moderate-poor | Moderate | -- | Good |
| S | Good | Good | -- | Good | Moderate | Moderate | -- | Good |
| D | Good | Good | Good | Good | Good | Moderate | Good | -- |
| T | Good | Good | Good | Good | Moderate | Moderate | Good | Good |
| A | Good | Good | Moderate | Good | Poor | Moderate | Poor | Good |
| C | Good | Good | Moderate-poor | Poor | Poor | Moderate | Poor | Good |

**[Table 2]**

| Colon segment | Group A | | Group B | | Group C | | | Group D |
|---|---|---|---|---|---|---|---|---|
| | Case 1 | Case 2 | Case 3 | Case 4 | Case 5 | Case 6 | Case 7 | Case 8 |
| R | Liquid | Gel | Liquid | Solid | Solid | Solid | -- | Gel |
| S | Liquid | Gel | -- | Solid | Solid | Gel | -- | Gel |
| D | Liquid | Liquid | Liquid | Solid | Gel | Gel | Liquid | -- |
| T | Liquid | Liquid | Liquid | Solid | Liquid | Gel | Liquid | Gel |
| A | Liquid | Gel | Solid | Liquid | Solid | Liquid | Solid | Gel |
| C | Liquid | Gel | Solid | Gel | Solid | Liquid | Solid | Gel |

Although the subjects of Group A only drank less than half of the conventional amount of 2 liters of the oral colonic irrigation agent, residue was favorably tagged by the contrast medium throughout the entire large intestine. Moreover, residue was in the form of a liquid or gel in all segments of the large intestine. FIG 1 shows a three-dimensional image (endoscope-like image) within the transverse colon (T) of a subject of Group A. In this manner, only polypoid lesions were observed as protrusions in the resulting three-dimensional images since solid residue was not present, and the images were able to be interpreted both easily and rapidly. Moreover, combining three-dimensional images captured in the supine and prone positions made it possible to observe the entire large intestine.

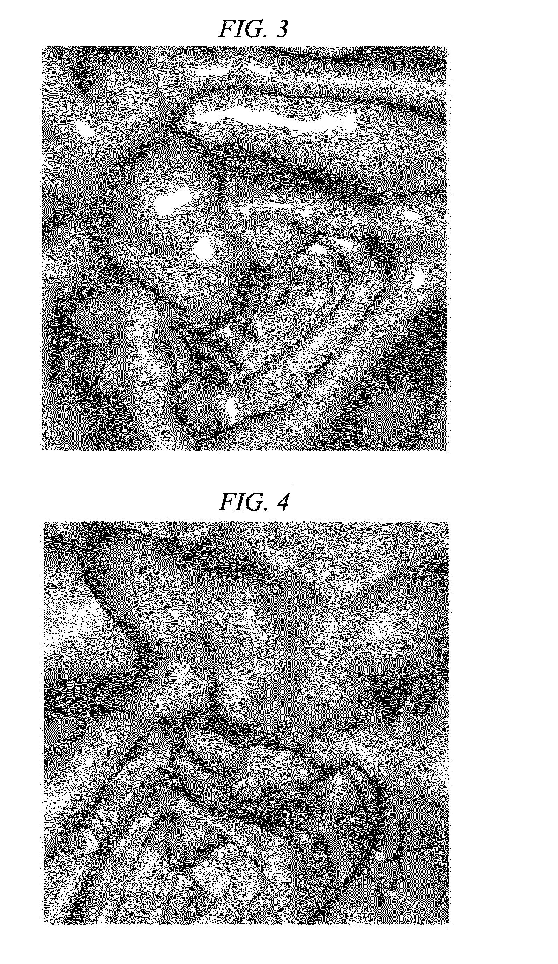

On the other hand, in Group B, in which an oral colonic irrigation agent was not taken, and in Group C, in which only half the amounts of Group A and D was taken, there were regions where the contrast medium and residue were not evenly mixed and regions where the contrast medium did not reach the residue resulting in poor tagging of residue by the contrast medium depending on the particular segment of the large intestine. The degree of tagging by the contrast medium was particularly low in the rectum (R), ascending colon (A) and cecum (C) of the large intestine in particular. This is presumed to be the result of the contrast medium having failed to mix uniformly with food ingested before and after pretreatment due to an inadequate amount of oral colonic irrigation agent.
In addition, the majority of the residue in Groups B and C was solid. FIGS. 2 and 3 show three-dimensional images (endoscope-like image) within the transverse colon (T) of subjects of Group B, while FIG. 4 shows a three-dimensional image (endoscope-like image) within the transverse colon (T) of a subject of Group C. These three-dimensional images indicate the inside of the transverse colon in respectively different subjects. In this manner, since a large amount of solid residue remained, polyp lesions were unable to be distinguished from this residue based on these three-dimensional images, thereby causing a decrease in test accuracy. In addition, since the resulting three-dimensional images were difficult to interpret, more time was required to interpret these images than in the case of Groups A and D.

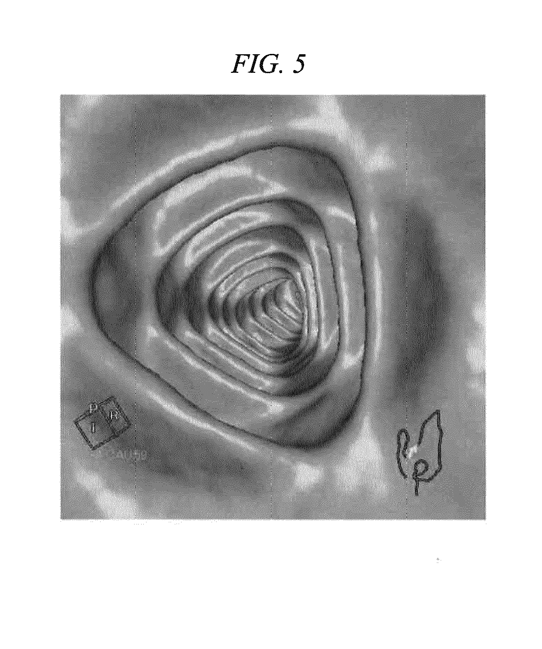

In addition, in group D, in which the subject took both the liquid preparation for oral administration of the present invention and a gastrointestinal function accelerator in the form of mosapride citrate, residue was favorably tagged by the contrast medium throughout the entire large intestine in the same manner as in Group A. Moreover, in addition to the residue being in the form of a gel in all segments of the large intestine, the amount of residue present was extremely small. This is presumed to be the result of excretion of residue in the large intestine having been accelerated during the time from the day before examination when pretreatment was started until the time of the examination. FIG. 5 shows a three-dimensional image (endoscope-like image) within the transverse colon (T) of a subject of Group D. In this manner, since there was hardly any residue present, the resulting three-dimensional images were able to be interpreted even more easily and rapidly than in Group A throughout the entire large intestine.

A questionnaire survey of subject acceptance was conducted following the examinations. All of the subjects of Groups A to D were able to lead normal daily lives, including work and diet, on the day pretreatment was performed (day before examination). In addition, their work and lives were not impaired by frequent bowel movements. During the night on the day pretreatment was performed (day before examination), the subjects were able to sleep through the night without waking up with the exception of Case 2 in Group A who woke up twice in order to have bowel movements (although the subject was able to sleep) and Case 4 in Group B who was up all night as a result of working at night (night shift). Moreover, none of the subjects were dissatisfied with the taste of the iodine-based water-soluble contrast medium they took during pretreatment. This is presumed to be the result of the nonionic iodine-based contrast medium used in the examples having a sweet taste without being bitter in contrast to ionic iodine-based contrast media having a potent bitter taste (such as Gastrografin™, trade name, Bayer Pharma AG, generic name: meglumine sodium amidotrizoate). In addition, there were no adverse side effects or harmful events observed accompanying pretreatment in any of the subjects of Groups A to D.

On the basis of these results, pretreatment consisting of taking a liquid preparation containing an iodine compound and a water-soluble polymer on the day before CT colonography in an amount that is equal to or less than the amount of oral colonic irrigation agent used for conventional colonic irrigation placed little burden on the subjects, was highly accepted by the subjects, was able to maintain a high level of depiction capacity comparable to that of the prior art, and clearly allowed the obtaining of adequate intestinal pretreatment effects required for accurate interpretation of the resulting images.

### [Example 2]

Images of the large intestine obtained by CT imaging were evaluated for subjects in Groups A to G (consisting of 2 subjects each) who had taken 300 mL to 450 mL of the liquid preparation for oral administration of the present invention on the day before CT colonography. The subjects of all groups took 20 mg of mosapride citrate (Gasmotin™, trade name, Dainippon Sumitomo Pharma Co., Ltd.) on two occasions consisting of after breakfast and after dinner on the day before the examination, after which they fasted and were only allowed to drink water from after dinner to completion of MDCT imaging. Moreover, the subjects took two sodium picosulfate tablets (Shinluck Tablets 2.5™, trade name, Iwaki Seiyaku Co., Ltd.) after dinner on the day before examination (5 mg as sodium picosulfate hydrate). However, Case 5 in Group C did not take either the mosapride citrate or sodium picosulfate.

Specific pretreatment of each group was as indicated below. Furthermore, among the two subjects in each group, one of the subjects took an ionic iodine-based contrast medium Gastrografin™, trade name, Bayer Pharma AG, iodine content: 370 mg/mL) for the iodine compound, while the remaining subject took a nonionic iodine-based contrast medium (Oypalomin 370 for Injection™, trade name, Fuji Pharma Co., Ltd., generic name: iopamidol, iodine content: 370 mg/mL). In addition, the total amount of liquid preparation for oral administration taken (mL), amount taken per single administration (mL), number of administrations (times), total amount of iodine compound used (total amount of iodine (mg)), and concentration of iodine compound in liquid preparation for oral administration taken (iodine concentration (mg/mL)) in each group are summarized in Table 3.

Group A: A total of 150 mL of a liquid preparation obtained by incorporating 20 mL of an iodine-based water-soluble contrast medium in 130 mL of an oral colonic irrigation agent (Niflec™ internal preparation, trade name, Ajinomoto Pharmaceuticals Co., Ltd.) (contrast agent concentration: 13.3% by volume) were taken internally on the day before the examination by dividing among three administrations consisting of after breakfast, after lunch and after dinner. The total amount taken in one day was 450 mL (consisting of 390 mL of oral colonic irrigation agent and 60 mL of iodine-based water-soluble contrast medium).

Group B: A total of 400 mL of a liquid preparation obtained by mixing 20 mL of an iodine-based water-soluble contrast medium with 380 mL of an oral colonic irrigation agent (Niflec™ internal preparation, trade name, Ajinomoto Pharmaceuticals Co., Ltd.) (contrast agent concentration: 10% by volume) were taken internally after dinner on the day before the examination. The total amount taken in one day was 400 mL (consisting of 380 mL of oral colonic irrigation agent and 20 mL of iodine-based water-soluble contrast medium).

Group C: A total of 200 mL of a liquid preparation obtained by incorporating 20 mL of an iodine-based water-soluble contrast medium in 180 mL of an oral colonic irrigation agent (Niflec™ internal preparation, trade name, Ajinomoto Pharmaceuticals Co., Ltd.) (contrast medium concentration: 10% by volume) were taken internally on the day before the examination by dividing among two administrations consisting of after breakfast and after dinner. The total amount taken in one day was 400 mL (consisting of 360 mL of oral colonic irrigation agent and 40 mL of iodine-based water-soluble contrast medium).

Group D: A total of 200 mL of a liquid preparation obtained by incorporating 40 mL of an iodine-based water-soluble contrast medium in 160 mL of an oral colonic irrigation agent (Niflec™ internal preparation, trade name, Ajinomoto Pharmaceuticals Co., Ltd.) (contrast agent concentration: 20% by volume) were taken internally on the day before the examination by dividing among two administrations consisting of after breakfast and after dinner. The total amount taken in one day was 400 mL (consisting of 320 mL of oral colonic irrigation agent and 80 mL of iodine-based water-soluble contrast medium).

Group E: A total of 150 mL of a liquid preparation obtained by incorporating 20 mL of an iodine-based water-soluble contrast medium in 130 mL of an oral colonic irrigation agent (Niflec™ internal preparation, trade name, Ajinomoto Pharmaceuticals Co., Ltd.) (contrast agent concentration: 13.3% by volume) were taken internally on the day before the examination by dividing among two administrations consisting of after breakfast and after dinner. The total amount taken in one day was 300 mL (consisting of 260 mL of oral colonic irrigation agent and 40 mL of iodine-based water-soluble contrast medium).

Group F: A total of 150 mL of a liquid preparation obtained by incorporating 45 mL of an iodine-based water-soluble contrast medium in 105 mL of an oral colonic irrigation agent (Niflec™ internal preparation, trade name, Ajinomoto Pharmaceuticals Co., Ltd.) (contrast agent concentration: 30% by volume) were taken internally on the day before the examination by dividing among two administrations consisting of after breakfast and after dinner. The total amount taken in one day was 300 mL (consisting of 210 mL of oral colonic irrigation agent and 90 mL of iodine-based water-soluble contrast medium).

Group G: A total of 100 mL of a liquid preparation obtained by incorporating 30 mL of an iodine-based water-soluble contrast medium in 70 mL of an oral colonic irrigation agent (Niflec™ internal preparation, trade name, Ajinomoto Pharmaceuticals Co., Ltd.) (contrast agent concentration: 30% by volume) were taken internally on the day before the examination by dividing among three administrations consisting of after breakfast, after lunch and after dinner. The total amount taken in one day was 300 mL (consisting of 210 mL of oral colonic irrigation agent and 90 mL of iodine-based water-soluble contrast medium).

**[Table 3]**

| Group | Total amount taken (mL) | Amount per administration (mL) | No. of times taken (times) | Total amount of iodine (mg) | Iodine concentration (mg/mL) |
|---|---|---|---|---|---|
| A | 450 | 150 | 3 | 22200 | 49 |
| B | 400 | 400 | 1 | 7400 | 18.5 |
| C | 400 | 200 | 2 | 14800 | 37 |
| D | 400 | 200 | 2 | 29600 | 74 |
| E | 300 | 150 | 2 | 14800 | 49 |
| F | 300 | 150 | 2 | 33300 | 111 |
| G | 300 | 100 | 3 | 33000 | 111 |

MDCT imaging was performed in the same manner as Example 1 on the day after taking the liquid preparation for oral administration of the present invention, and two types of three-dimensional images (endoscope-like images and enema-like images combining images of liquid residue and gas) were constructed. In addition, two-dimensional images in the form of axial images were constructed, and the degree of residue tagging by the contrast medium and the physical state of the residue were compared among each group, followed by an evaluation of the quality of intestinal pretreatment using each of the methods. Residue was evaluated by dividing the large colon into six sections consisting of the cecum (C), ascending colon (A), transverse colon (T), descending colon (D), sigmoid colon (S) and rectum (R). Tables 4 to 17 show the results of evaluating residue in each case. In Tables 4 to 17, "HUmax" indicates the maximum value of the CT value in each segment, while "HUmin" indicates the minimum value of the CT value in each segment. In addition, in the residue pattern columns, "SC" is the abbreviation for "scattered", indicating a sporadic scattering of a small amount of residue.

**[Table 4]**

| Group A - Case 1 (Ionic Iodine-Based Contrast Medium) | | | | | | |
|---|---|---|---|---|---|---|
| Colon Segment | Liquid Residue | Solid Residue | Tagging | HUmax | HUmin | Residue Pattern |
| R | Low | Low | Good | 760 | 512 | Liquid,SC |
| S | Low | Low | Good | 964 | 346 | SC |
| D | Moderate | None | Good | 1124 | 803 | Liquid |
| T | Moderate | None | Good | 1097 | 899 | Liquid |
| A | Moderate | None | Good | 1023 | 792 | Liquid |
| C | Moderate | None | Good | 999 | 575 | Liquid |

**[Table 5]**

| Group A - Case 2 (Nonionic Iodine-Based Contrast Medium) | | | | | | |
|---|---|---|---|---|---|---|
| Colon Segment | Liquid Residue | Solid Residue | Tagging | HUmax | HUmin | Residue Pattern |
| R | Moderate | Low | Good | 1575 | 1303 | Liquid,SC |
| S | Low | Low | Good | 1552 | 169 | Liquid,SC |
| D | Low | Low | Good | 1567 | 427 | Liquid,SC |
| T | Moderate | Low | Good | 1593 | 297 | Liquid,SC |
| A | None | Moderate | Somewhat poor | 1371 | 466 | Gel,SC |
| C | None | Moderate | Somewhat poor | 1578 | 1017 | Gel,SC |

**[Table 6]**

| Group B - Case 3 (Ionic Iodine-Based Contrast Medium) | | | | | | |
|---|---|---|---|---|---|---|
| Colon Segment | Liquid Residue | Solid Residue | Tagging | HUmax | HUmin | Residue Pattern |
| R | None | None | -- | -- | -- | -- |
| S | None | None | -- | -- | -- | -- |
| D | None | None | -- | -- | -- | -- |
| T | Low | Low | Good | 1066 | 574 | Liquid,Gel |
| A | Low | Low | Good | 1030 | 885 | Liquid,SC |
| C | None | Low | Good | 959 | 795 | Gel,SC |

**[Table 7]**

| Group B - Case 4 (Nonionic Iodine-Based Contrast Medium) | | | | | | |
|---|---|---|---|---|---|---|
| Colon Segment | Liquid Residue | Solid Residue | Tagging | HUmax | HUmin | Residue Pattern |
| R | None | None | -- | -- | -- | -- |
| S | Low | None | Good | 826 | 651 | Liquid |
| D | Low | None | Good | 698 | 231 | Liquid |
| T | Low | None | Good | 796 | 593 | Liquid |
| A | Low | None | Good | 856 | 637 | Liquid |
| C | Low | None | Poor | 544 | -31 | Liquid |

**[Table 8]**

| Group C - Case 5 (Ionic Iodine-Based Contrast Medium) | | | | | | |
|---|---|---|---|---|---|---|
| Colon Segment | Liquid Residue | Solid Residue | Tagging | HUmax | HUmin | Residue Pattern |
| R | Moderate | None | Good | 1107 | 673 | Liquid |
| S | Low | Low | Good | 996 | 746 | Liquid,SC |
| D | Low | None | Good | 873 | 773 | Liquid |
| T | Moderate | None | Good | 909 | 667 | Liquid |
| A | Low | None | Good | 559 | 455 | Liquid |
| C | Low | None | Poor | 256 | -8 | Liquid |

**[Table 9]**

| Group C - Case 6 (Nonionic Iodine-Based Contrast Medium) | | | | | | |
|---|---|---|---|---|---|---|
| Colon Segment | Liquid Residue | Solid Residue | Tagging | HUmax | HUmin | Residue Pattern |
| R | None | None | - | - | - | - |
| S | None | Low | Good | 1194 | 125 | Gel,SC |
| D | None | None | -- | -- | -- | -- |
| T | None | Low | Good | 990 | 201 | Gel,SC |
| A | None | High | Good | 1235 | 701 | Gel,SC |
| C | None | High | Good | 1024 | 178 | Gel,SC |

**[Table 10]**

| Group D - Case 7 (Ionic Iodine-Based Contrast Medium) | | | | | | |
|---|---|---|---|---|---|---|
| Colon Segment | Liquid Residue | Solid Residue | Tagging | HUmax | HUmin | Residue Pattern |
| R | None | None | -- | -- | -- | -- |
| S | None | Moderate | Good | 895 | 342 | Gel,SC |
| D | None | Low | Good | 850 | 545 | Gel |
| T | None | Moderate | Good | 543 | 380 | Gel |
| A | Low | None | Somewhat poor | 239 | 86 | Liquid |
| C | None | None | -- | -- | -- | -- |

**[Table 11]**

| Group D - Case 8 (Nonionic Iodine-Based Contrast Medium) | | | | | | |
|---|---|---|---|---|---|---|
| Colon Segment | Liquid Residue | Solid Residue | Tagging | HUmax | HUmin | Residue Pattern |
| R | None | Low | Good | 374 | 175 | SC |
| S | None | None | -- | -- | -- | -- |
| D | None | Low | Good | 576 | 348 | SC |
| T | None | Low | Somewhat poor | 416 | 53 | SC |
| A | Low | None | Poor | 220 | 50 | Liquid |
| C | Low | None | Poor | 97 | 9 | Liquid |

**[Table 12]**

| Group E - Case 9 (Ionic Iodine-Based Contrast Medium) | | | | | | |
|---|---|---|---|---|---|---|
| Colon Segment | Liquid Residue | Solid Residue | Tagging | HUmax | HUmin | Residue Pattern |
| R | None | None | - | - | - | - |
| S | None | None | -- | -- | -- | -- |
| D | None | None | -- | -- | -- | -- |
| T | None | None | -- | -- | -- | -- |
| A | None | Low | Poor | 57 | -68 | Gel |
| C | None | Moderate | Poor | 87 | -10 | Gel |

**[Table 13]**

| Group E - Case 10 (Nonionic Iodine-Based Contrast Medium) | | | | | | |
|---|---|---|---|---|---|---|
| Colon Segment | Liquid Residue | Solid Residue | Tagging | HUmax | HUmin | Residue Pattern |
| R | None | Moderate | Good | 1353 | 287 | Solid,SC |
| S | None | Moderate | Good | 1344 | 596 | Solid,SC |
| D | None | Low | Good | 548 | 351 | Solid,SC |
| T | None | Moderate | Good | 718 | 435 | Solid,SC |
| A | None | Moderate | Good | 730 | 473 | Solid,SC |
| C | None | None | -- | -- | -- | -- |

**[Table 14]**

| Group F - Case 11 (Ionic Iodine-Based Contrast Medium) | | | | | | |
|---|---|---|---|---|---|---|
| Colon Segment | Liquid Residue | Solid Residue | Tagging | HUmax | HUmin | Residue Pattern |
| R | Low | None | Good | 658 | 456 | Liquid |
| S | Low | None | Good | 631 | 415 | Liquid |
| D | Low | None | Good | 844 | 378 | Liquid |
| T | Low | None | Good | 800 | 544 | Liquid |
| A | Low | None | Good | 647 | 235 | Liquid |
| C | Low | None | Good | 492 | 232 | Liquid |

**[Table 15]**

| Group F - Case 12 (Nonionic Iodine-Based Contrast Medium) | | | | | | |
|---|---|---|---|---|---|---|
| Colon Segment | Liquid Residue | Solid Residue | Tagging | HUmax | HUmin | Residue Pattern |
| R | None | Low | Good | 1047 | 628 | Gel,SC |
| S | None | Moderate | Good | 1576 | 693 | Gel,SC |
| D | None | High | Good | 1685 | 636 | Gel,SC |
| T | None | Moderate | Good | 1245 | 844 | Gel,SC |
| A | None | None | -- | -- | -- | -- |
| C | None | None | -- | -- | -- | -- |

**[Table 16]**

| Group G - Case 13 (Ionic Iodine-Based Contrast Medium) | | | | | | |
|---|---|---|---|---|---|---|
| Colon Segment | Liquid Residue | Solid Residue | Tagging | HUmax | HUmin | Residue Pattern |
| R | None | None | -- | -- | -- | -- |
| S | None | Low | Good | 1025 | 206 | SC |
| D | None | Low | Good | 1104 | 486 | Gel |
| T | Moderate | None | Good | 1291 | 695 | Liquid |
| A | Moderate | None | Good | 1179 | 952 | Liquid |
| C | Low | None | Good | 791 | 296 | Liquid |

**[Table 17]**

| Group G - Case 14 (Nonionic Iodine-Based Contrast Medium) | | | | | | |
|---|---|---|---|---|---|---|
| Colon Segment | Liquid Residue | Solid Residue | Tagging | HUmax | HUmin | Residue Pattern |
| R | None | Low | Good | 633 | 366 | Gel |
| S | None | None | -- | -- | -- | -- |
| D | None | None | -- | -- | -- | -- |
| T | Low | None | Good | 952 | 857 | Liquid |
| A | Low | None | Good | 972 | 611 | Liquid |
| C | None | None | -- | -- | -- | -- |

Although there were cases in which tagging was somewhat poor in certain segments of the colon (especially in the cecum (C) and ascending colon (A)), the majority of the colon was tagged favorably in all cases and images were able to be interpreted. In addition, although there were cases in which solid residue was present, the residue was able to be favorably tagged in each case, and since the amount of residue was high, the solid residue was distinguished comparatively easily from protrusions in the intestine such as polyp lesions. In Group G in particular, despite the amount taken in a single administration being only 100 mL and the total amount taken being extremely low at only 300 mL, the amount of solid residue throughout the entire colon was extremely low, tagging was good, and interpretation of the resulting images was extremely easy. In addition, residue was tagged favorably in the same manner as other cases and images were able to be interpreted even in Case 5 of Group C that did not take either mosapride citrate or sodium picosulfate.

### INDUSTRIAL APPLICABILITY

The liquid preparation for oral administration of the present invention can be favorably used for pretreatment of CT colonography, and can be used in colon examinations and other clinical testing fields, and particularly for group screening and other forms of primary screening.

## Claims

1. A liquid preparation for oral administration taken during gastrointestinal imaging by CT colonography, comprising:
an iodine compound and a water-soluble polymer or saline laxative, wherein
300 mL to 1200 mL are taken on the day before CT colonography in a single administration or divided among several administrations.

2. The liquid preparation for oral administration according to claim 1, wherein 600 mL to 1200 mL are taken on the day before CT colonography in a single administration or divided among several administrations.

3. The liquid preparation for oral administration according to claim 1 or 2, wherein the iodine compound is nonionic.

4. The liquid preparation for oral administration according to any one of claims 1 to 3, wherein the water-soluble polymer is one or more selected from the group consisting of polyethylene glycol, polydextrose, dextran, dextrin, hydroxyethyl starch, gum arabic, pullulan, pectin, albumin and carboxymethyl cellulose.

5. The liquid preparation for oral administration according to any one of claims 1 to 4, wherein osmotic pressure is 200 mOsm/L to 440 mOsm/L.

6. The liquid preparation for oral administration according to any one of claims 1 to 5, further comprising one or more of sugars selected from the group consisting of sucrose, sorbitol, xylitol, erythritol, mannitol, trehalose, lactitol, lactulose, maltitol, palatinose, raffinose and glycerin.

7. The liquid preparation for oral administration according to any one of claims 1 to 6, wherein the content of the iodine compound is 3.5 mg/mL to 90 mg/mL.

8. The liquid preparation for oral administration according to any one of claims 1 to 7, further comprising one or more of compounds selected from the group consisting of dimethylpolysiloxane and gastrointestinal function accelerators.

9. A composition for gastrointestinal radiography, comprising:
an iodine compound and a water-soluble polymer or saline laxative, wherein
300 mL to 1200 mL are taken in the form of an aqueous solution, having an iodine content of 3.5 mg/mL to 90 mg/mL prepared by dissolving in water, on the day before CT colonography in a single administration or divided among several administrations.

10. The composition for gastrointestinal radiography according to claim 9, wherein 600 mL to 1200 mL are taken in the form of an aqueous solution, having an iodine content of 3.5 mg/mL to 90 mg/mL prepared by dissolving in water, on the day before CT colonography in a single administration or divided among several administrations.

11. The composition for gastrointestinal radiography according to claim 9 or 10, wherein the iodine compound is nonionic.

12. The composition for gastrointestinal radiography according to any one of claims 9 to 11, comprising:
electrolytes such that the osmotic pressure when dissolved in water is 200 mOsm/L to 440 mOsm/L, and
one or more of water-soluble polymers selected from the group consisting of polyethylene glycol, polydextrose, dextran, dextrin, hydroxyethyl starch, gum arabic, pullulan, pectin, albumin and carboxymethyl cellulose, or
one or more of saline laxatives selected from the group consisting of magnesium citrate, sodium dihydrogen phosphate and disodium hydrogen phosphate.

13. The composition for gastrointestinal radiography according to any one of claims 9 to 12, further comprising one or more of compounds selected from the group consisting of dimethylpolysiloxane and gastrointestinal function accelerators.

14. A pharmaceutical agent for pretreatment for CT colonography, comprising:
combining an iodine compound and a water-soluble polymer or saline laxative, wherein
300 mL to 1200 mL are taken on the day before CT colonography in a single administration or divided among several administrations.

15. The pharmaceutical agent for pretreatment for CT colonography according to claim 14, wherein 600 mL to 1200 mL are taken on the day before CT colonography in a single administration or divided among several administrations.

16. A pharmaceutical agent for pretreatment for CT colonography, comprising:
combining an oral colonic irrigation agent and an iodine-containing contrast agent, wherein
300 mL to 1200 mL are taken on the day before CT colonography in a single administration or divided among several administrations.

17. The pharmaceutical agent for pretreatment for CT colonography according to claim 16, wherein 600 mL to 1200 mL are taken on the day before CT colonography in a single administration or divided among several administrations.
